# EUROPEAN PATENT SPECIFICATION

(11) **EP 2 101 672 B1**
(45) Date of publication and mention of the grant of the patent: **22.08.2012**
(21) Application number: 08713482.1
(22) Date of filing: 03.01.2008
(51) Int. Cl.: A61F 9/008

(54) **LASER BEAM ALIGNMENT DEVICES, SYSTEMS, AND METHODS**
VORRICHTUNGEN, SYSTEME UND VERFAHREN ZUR AUSRICHTUNG VON LASERSTRAHLEN
DISPOSITIFS, SYSTÈMES ET PROCÉDÉS D'ALIGNEMENT DE FAISCEAU LASER

(30) Priority: 05.01.2007 US 620493
(43) Date of publication of application: 23.09.2009
(73) Proprietor: AMO MANUFACTURING USA, INC., Milpitas, CA 95035 (US)
(72) Inventor: GRAY, Michael, S., Sunnyvale, CA 94085 (US)
(74) Representative: HOFFMANN EITLE
(86) International application number: PCT/US2008/050137
(87) International publication number: WO 2008/086111

(56) References cited:
- EP-A- 1 273 279
- WO-A-99/24796
- US-A- 5 684 593
- US-A1- 2005 021 011
- US-A1- 2006 092 406

## Description

### Background of the Invention

### Field of the Invention

This invention relates generally to laser beam delivery systems and methods of making and using such systems. More specifically the invention relates to devices and methods for aligning and affecting the performance of laser beam delivery systems.

### Description of the Related Art

Laser beam delivery systems are used to direct and/or condition the energy or power in pulsed and continuous lasers so that the resulting beam can perform a desired function. For example, in a laser vision correction (LVC) procedure (e.g., a PRK or LASIK procedure), a laser beam from a pulsed laser is precisely directed and/or focus at an ablation plane in which the cornea of a subject is located. A laser beam delivery system is used to condition the beam and to scan the beam across the corneal surface in order to ablate small portions of the cornea in a very controlled manner.

Because of the precision required in performing an LVC procedure, it is critical that the laser be properly aligned to the ablation plane. In addition, the delivery system may also be aligned to precisely direct portions of the beam into monitoring equipment used during the procedure. In practice, such laser beam delivery systems are relatively complex, and alignment or realignment can be very time consuming. In some cases, it may also be necessary to inspect the beams cross-sectional profile. For example, because of the relatively high beam energies involve, as well as for other reasons, the quality of the beam may degrade over time, for example, due to damage to optical components within the system and/or laser cavity (e.g., the laser output coupler).

Accordingly, devices and methods are needed that will reduce the time required to precisely align laser beam delivery systems and to insure that the laser output maintains a desired quality.

A tool according to the preamble of claim 9 is taught by US2006/0092406.

### Summary of the Invention

The present invention is generally directed to devices, systems, and methods for aligning laser beam delivery systems, as defined in independent claims 1, 6 and 9, for example, laser delivery systems for use in ophthalmic procdures such as laser vision correction procedures. One aspect of the present invention involves an alignment tool for a laser beam delivery system, comprising a base including one or more registration elements and an alignment optic operably coupled to the base. The alignment optic comprises a fluorescent element and a front surface. The fluorescent element is configured to fluoresce when illuminated by electromagnetic radiation produced by a laser. The front surface comprises a reflective surface and an alignment mark. The reflective surface is configured to specularly reflect the electromagnetic radiation, while the alignment mark, which is centrally disposed within the reflective surface, is configured to diffusely scatter the electromagnetic radiation.

In another aspect of the invention, a laser beam delivery system for an ocular surgery comprises a laser configured to produce a beam of electromagnetic radiation, a mirror, an alignment optic, a fluorescent element, and a site configured to removably receive either one of the mirror and the alignment optic. The alignment optic comprises a fluorescent element configured to fluoresce when illuminated by electromagnetic radiation produced by the laser. The alignment optic further comprises a front surface that includes a reflective surface and an alignment mark. The reflective surface is configured to specularly reflect at least a portion of the laser beam. The alignment mark is centrally disposed within the reflective surface and is configured to diffusely scatter at least some of the electromagnetic radiation of the laser beam. The system is configured so that at least a portion of the laser beam reflects in a predetermined direction, regardless of whether the alignment optic or the mirror is disposed at the site.

In yet another aspect of the invention, a method of aligning a laser beam delivery system for an ocular surgery comprises providing a laser beam, a first mirror, and an alignment tool. The alignment tool comprises a fluorescent element configured to fluoresce when illuminated by electromagnetic radiation produced by the laser and a front surface according to an embodiment of the invention. The method further comprises mounting the alignment tool at a location and directing the laser beam onto the alignment tool so as to cause a first portion of the radiation in the laser beam to diffusely scatter and a second portion of the radiation in the laser beam to specularly reflect. The method additionally comprises, in response to the reflected portion, adjusting the alignment tool until the portion of the laser beam is reflected in a predetermined direction from the first location. The also comprises removing the alignment tool and mounting the first mirror at the first location so that the laser beam is reflected in the predetermined direction by the mirror.

### Brief Description of the Drawings

Embodiments of the present invention may be better understood from the following detailed description when read in conjunction with the accompanying drawings. Such embodiments, which are for illustrative purposes only, depict the novel and non-obvious aspects of the invention. The drawings include the following figures:
FIG. 1 is a schematic of a laser beam delivery system.
FIG. 2 is a perspective view of an alignment tool according to an embodiment of the invention.
FIG. 3 is a front view of the alignment tool shown in FIG. 2
FIG. 4 is a front view of an alignment tool according to a second embodiment of the invention.
FIG. 5 is a front view of an alignment tool according to a third embodiment of the invention.
FIG. 6 is a front view of an alignment tool according to a fourth embodiment of the invention.
FIG. 7 is a front view of an alignment tool according to a fifth embodiment of the invention.
FIG. 8 is a sectional view of an alignment tool shown in FIG. 7.
FIG. 9 is perspective view of a turning mirror and associated mount from a laser beam delivery system according to an embodiment of the invention.
FIG. 10 is a block diagram of a method of aligning or calibrating a laser beam delivery system according to an embodiment of the invention.
FIG. 11 is a top view of alignment tool shown in FIG. 2, in which a first portion of a laser beam is specularly deflected and a second portion of the laser beam is diffusely scattered.
FIG. 12 is a top view of the alignment tool shown in FIG. 11 after the orientation of the alignment tool has been adjusted.
FIG. 13 is a top view of the mirror and associated mount shown in FIG. 9 after use of the alignment tool shown in FIG. 2.

### Detailed Description of the Drawings

Embodiments of the present invention are generally directed to laser beam delivery systems and to devices, systems, and methods for aligning and affecting the performance thereof. Embodiments of the invention may find particular utility in the alignment of laser beam delivery systems configured for performing surgical procedures, for example, for performing ocular surgical procedures such as a laser vision correction (LVC) procedure (e.g., a LASIK, LASEK, or PRK procedure) to provide ametropia, or a radial femtosecond lensotomy of a presbyopic lens to increase lens malleability and accommodative capability. In an exemplary embodiment, an alignment tool, along with methods of use thereof, is discussed that may be used to efficiently and accurately align an ocular surgical system suitable for performing an LVC procedure.

Embodiments of the invention may be used with lasers in visible, ultra-violet (UV), infrared (IR), or near infrared (NIR) waveband range of the electromagnetic spectrum. The laser may produce a continuous beam or a beam comprising a series of discreet pulses. The laser may be an excimer laser, YAG laser, solid-state or fiber laser, Argon laser, diode laser, HeNe lasers, and the like. Lasers producing pulses in the nano-second, pico-second, and femto-second range may find particular use in ocular and other medical applications, which are known to reduce tissue damage that can be caused by heat. Such lasers include, but are not limited to, excimer lasers, neodymium:YAG (Nd:YAG), and Nd:YLF. Examples of such lasers include the Coherent RegA 9000/9050 Ti:Sapphire available from Coherent Inc. (Santa Clara, CA, USA), the IMRA FCPA microjoule D-1000 Ytterbium fiber oscillator/amplifier laser system available from IMRA America Inc. (Ann Arbor, MI, USA), the high-repetition rate, cavity dumped, mode-locked ultrafast femtoNOVA available from High Q Laser Production GmbH (Hohenems, Austria), or the neodymium:YLF (Nd:YLF) laser ISL Model 2001 MPL or 4001 CLS from Intelligent Laser Systems, Inc. of San Diego, Calif.

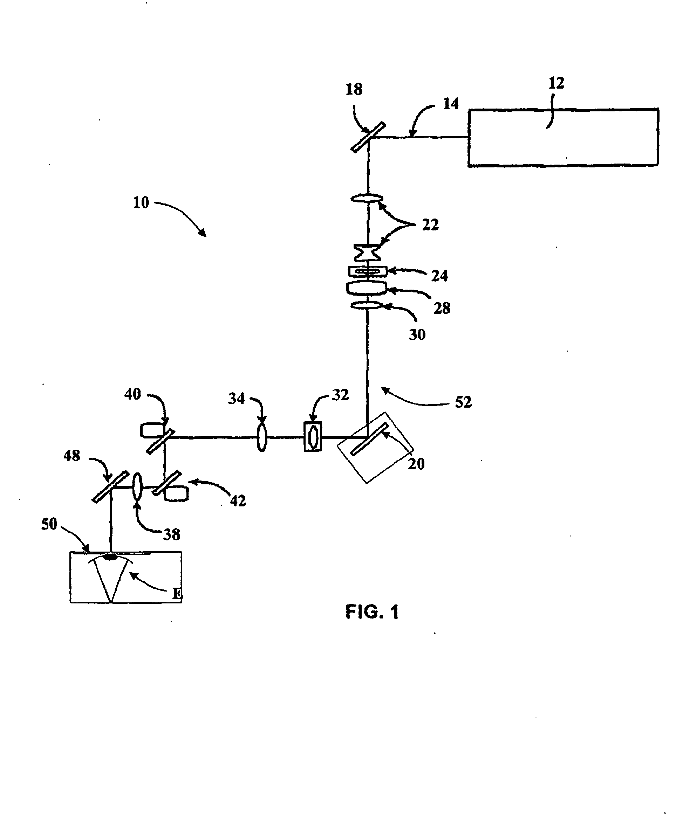

Referring to FIG. 1, in an illustrative embodiment, a laser system 10, for use in an ocular surgery, comprises a laser 12 that produces a laser beam 14 of electromagnetic radiation that is directed toward an eye E of a subject. The system 10 is configured to provide an LVC procedure in which corneal material of the eye E is ablated in order to provide a predetermined visual correction. The laser beam 14 is first directed towards a first turning mirror 18, which is generally disposed to direct the beam 14 toward a second turning mirror 20 after passing through a sizing telescope 22 that may be configured to size the beam 14 to a desired cross-sectional size. In one embodiment, the cross sectional area of the sized beam 14 is about 18 mm by about 20 mm upon exiting the sizing telescope 22. A lenslet aperture 24 is placed in front of a lenslet array 28 to condition and/or resize the beam 14, for example, to provide a substantially square beam with a cross-section of about 18 mm by about 18 mm. The lenslet array 28 is generally configured to produce a plurality of beamlets that may be configured to smooth or otherwise condition the beam 14 for use on the eye E. The aperture 24 is generally disposed so as to align the beam 14 to the lenslet array 28. A positive power re-focusing lens 30 is disposed after the lenslet array 28 and may be used to tilt the beamlets formed by the array 28 and/or to overlap the beamlets at an iris aperture 32, where the beamlets arrive after reflection by the second turning mirror 20.

In the illustrated embodiment, the beamlets pass through additional imaging lenses 34, 38 and mirrors 40, 42, 48, before reaching an ablation plane 50. In some embodiments, one or more of the mirrors 40, 42, 48 are configured as scanning mirrors that scan the beam 14 across the ablation plane 50 in a predetermined manner. It will be appreciated that other configuration may be used for conditioning and directing the beam 14 to the eye E and/or to other system components, for example, a beamsplitter. Also, while elements of the illustrated embodiment, such as the lenslet array 28, may be incorporated into ocular ablation systems with certain types of lasers, embodiments of the invention extend to other ocular and non-ocular laser beam delivery systems that do not use some or all of these elements. In general, laser beam delivery systems, devices, and methods according to embodiments of the invention will at least incorporate first and second turning mirrors 18, 20 for directing the beam 14 to a target location. The mirrors 18, 20, 40, 42, and 48 generally define a beam path 52 along which electromagnetic radiation from the laser 12 is directed to the ablation plane 50.

The laser 12 may comprise, but is not limited to, an excimer laser, which has been found to be provide favorable characteristics for ablating corneal material. For example, the laser 12 may comprise an argon-fluoride laser producing a plurality of pulses of laser light with a wavelength of approximately 193 nm and fixed pulse having a full width half maximum (FWHM) duration of about 15 nano seconds during a treatment. The laser 12 may be designed to provide a feedback stabilized fluence at the patient's eye, delivered via delivery optics. Embodiments of the present invention may also be useful with alternative sources of visible, ultraviolet, or infrared radiation, particularly those adapted to controllably ablate a corneal tissue without causing significant damage to adjacent and/or underling tissues of the eye. The laser system 10 may include, but is not limited to, other solid state lasers, for example, a frequency multiplied solid state laser such as flash-lamp and diode pumped solid state lasers. Exemplary solid state lasers for ophthalmic applications includes UV solid state lasers (approximately 193-215 nm) such as those described in U.S. Pat. Nos. 5,144,630 and 5,742,626, Borsuztky et al., "Tunable UV Radiation at Short Wavelengths (188-240 nm) Generated by Sum Frequency Mixing in Lithium Borate", Appl. Phys. 61:529-532 (1995), and the like.

The lenslet array 28 generally comprises a plurality of lenslets disposed in an n by m grid. In some embodiments the lenslets are configured in a square array, for example, a 4 by 4 grid having a total of 16 lenslets. The grid may be larger than 4 by 4, for example, 5 by 5, 6 by 6, 7 by 7, 8 by 8, or greater than 8 by 8. In certain embodiments, the lenslet array 28 may be rotated about an axis between different pulses of the laser beam 14. For example, the array 28 may be rotated approximately 45 degrees away from the 0 degrees and 90 degrees angles relative to the axis of the laser beam 14. In some embodiments, the angles of rotation of the array 28 arse within a window of 45 degrees ±10 degrees. Rotation may occur in 90 degrees increments between each pulse of a pulsed laser source 12, and with each rotation being within a window ±10 degrees. For example, angles of rotation could be about 45 degrees, 135 degrees, 225 degrees and 315 degrees. Such rotation helps prevent formation of a laser beam having areas of striping or a grid pattern caused by the areas of the lenslet array 28 between lenslets. Rotation may be achieved via a drive mechanism coupled with the lenslet array 28 or via any other suitable means.

In some embodiments, the lenslet array 28 and the re-focusing lens 40 may be combined in one optic. This may be achieved, for example, by a lenslet array 28 having a gradual curvature along one or both of its opposed surfaces to give the lenslet array 28 an overall low positive power lens effect. Such a combined optic is adapted to split the laser beam 14 into multiple beamlets and also make the beamlets overlap in the plane of the aperture 32. In various embodiments, such a curved lenslet array 28 could be formed by programming lithography software to give a slight curvature to the optic or by disposing the partial cylindrical elements of the lenslet 28 on a curved substrate.

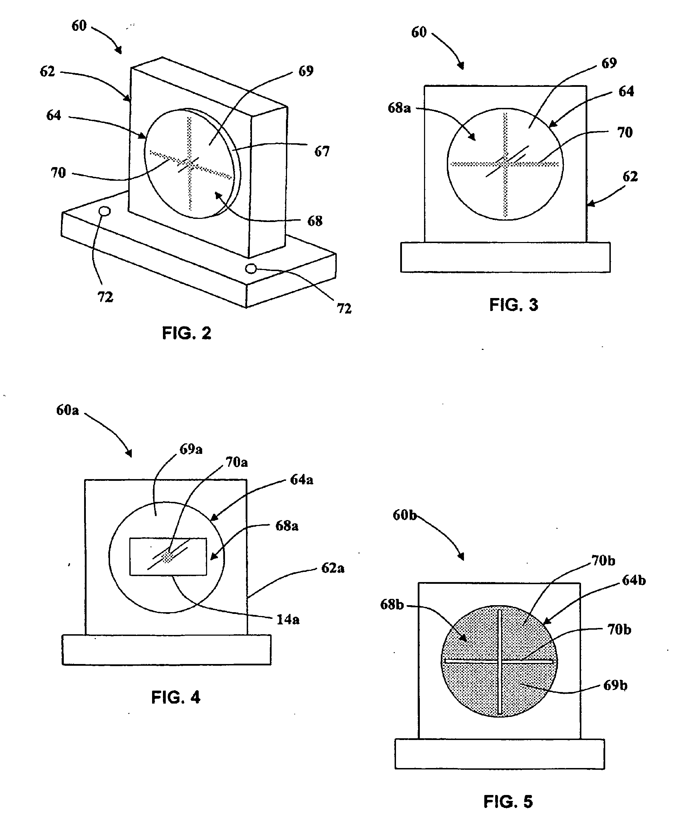

Referring to FIGS. 2 and 3, in certain embodiment, alignment of the laser beam delivery system 10 is accomplished, at least in part, using an alignment tool 60. The alignment tool comprises a fixture, mount, or base 62 and an alignment optic 64 operably coupled to the base 62. The alignment optic 64 comprises a fluorescent portion 67 made of a material that fluorescence in the presence of electromagnetic radiation produced by the laser 12, which is typically is in the ultra-violet wavelength range. The alignment optic 64 further comprises a front face or surface 68 that includes a reflective portion or surface 69 and an alignment mark 70 that is centrally disposed within the reflective surface 69. The front surface 68 may be made of the same material as the fluorescent portion 67. Alternatively, at least a portion of the front surface 68 may comprise a separate material that is disposed on top of the fluorescent portion 67. The reflective surface 69 is configured to specularly reflect the electromagnetic radiation from the laser beam 14, while the alignment mark 70 is configured to diffusely scatter the electromagnetic radiation incident thereon from the laser beam 14. The alignment mark 70 may be etched into the front surface 68 or otherwise formed to produce a surface texture appropriate for scattering electromagnetic radiation from the laser beam 14.

In general, the amount of laser 12 radiation scattered by the alignment mark 70 may be characterized by a scattering cross-section. In some embodiments, the alignment mark 70 is configured to scatter an amount of radiation that is at least twice the amount of radiation scattered by the reflective surface 69, more preferably at least 4 times the amount of radiation scattered by reflective surface 69, and even more preferably 10 times the amount of radiation scattered by reflective surface 69. In some embodiments, the amount of radiation scattered by the alignment mark 70 is determined by reflecting a beam off at least a portion of the alignment mark 70 of the alignment optic 64, and then measuring the amount of radiation received by a photodetector located on an axis of specular reflection from the alignment mark 70 surface. The photodetector may be disposed, for example, 10 centimeter to 1 meter or more from the alignment mark 70 and have a predetermined area. The amount of radiation received by the photodetector from the reflected beam may then be compared to the amount of radiation received by the photodetector under a reference condition. For example, the alignment optic 64 may be translated along the front surface 68 so that the beam is reflected off of the reflective surface 69. Alternatively, the alignment optic 64 may be removed and replaced by a reference surface that is optically smooth (e.g., having an rms roughness of less than about 10 nanometers, preferably less than 1 nanometer) and highly reflective to radiation in the beam. Preferably, the ratio of the radiation received by the detector in the presence of the alignment mark 70 compared to the radiation received by the detector in the presence of the reference surface is less than about 0.5, more preferably less than about 0.25, and even more preferably less than about 0.1.

The base 62 includes one or more registration elements 72 for placing the alignment tool 60 in the system 10 with a predetermined orientation and or position (e.g., at a predetermined angle and spatial location relative to the laser beam 14 from the laser 12). The registration elements 72 are generally configured to match corresponding registration elements of one or more mirror fixtures or bases that are part of the laser beam deliver system 10 used during normal operation thereof. In the illustrated embodiment, the registration elements 72 comprise two holes that are sized to receive mating pins (not shown), for example, located on a receiving base plate, translation stage, and/or rotation stage, or on a breadboard or table to which one or more components of the system 10 are attached. Alternatively, other registration elements and method known in the art may be used for providing a predetermined registration of the alignment tool 60 with the system 10 and with the laser beam 14. For example, one or both of the registration elements 72 may comprise a straight edge or groove that is precisely disposed so as to provide a desired geometric relationship between the alignment tool 60 and the system 10 and/or the laser beam 14. In some embodiments, the registration elements 72 may be configured or arranged to key the alignment tool 60 so it can only be install in one way.

In certain embodiments, the alignment mark 70 is a region of the front surface 68 that does not reflect radiation in the beam 14 along the beam path 52. It has been found that the resulting void in the beam 14 after reflection off the alignment optic 64 may be advantageously used during alignment of the system 10, for example, to align the beam 14 to another element in the system, such as a mirror or beamsplitter, or to one or more alignment pinholes used during alignment of the system. In the illustrated embodiment, the alignment mark 70 comprises a cross-hair 73 pattern that includes a horizontal bar or line 73a and an intersecting vertical bar or line 73b. It will be appreciated that the lines 73a, 73b may be rotated from horizontal/vertical orientations in the illustrated embodiment and/or may be configured to have a non-orthogonal relationship to one another.

Referring to FIG. 4, in some embodiments, an alignment tool 60a comprises a front surface 68a having reflective surface 69a and an alignment mark 70a that is circular or oval in shape and is disposed at or near the center of the front surface 68a. Alternatively, the alignment mark 70a may be some other shape, such as a square, rectangle, or triangle, which is relatively small and is disposed at or near the center of the front surface 68a. The use of such a small alignment mark 70a in the center of the front surface 68a may be useful, for example, in producing a small void in the center of the beam 14 that may be aligned to be coaxial with an alignment pinhole or system iris. The diameter or area of the alignment mark 70a is generally much smaller than the diameter or area of the reflective surface and/or smaller than a footprint or cross-section 14a of the laser beam 14. For example, the diameter of the reflective surface 69a may be at least about 5 times greater that the diameter of the alignment mark 70a (or the area of the reflective surface 69a may be at least about 25 times greater that the area of the alignment mark 70a). In other embodiments, the diameter of the reflective surface 69a is at least 10 times, or even 20 times or more, greater that the diameter of the alignment mark 70a. Similarly, in other embodiments, the diameter of the reflective surface 69a is at least 5 times, 10 times, or 20 times greater that the thickness of the lines 73a, 73b of the cross-hair 73. In general, alignment marks according to embodiments of the invention (e.g., alignment marks 70, 70a) are disposed such that the laser beam footprint 14a is substantially symmetrically disposed about at least one axis passing through the center of the alignment mark.

Referring to FIG. 5, in certain embodiments, an alignment optic 64b comprises a front surface 68b, which includes a diffuse portion or surface 69b that is configured to diffusely scatter electromagnetic radiation from the laser beam 14, and an alignment mark 70b, which is configured to specularly reflect the laser beam 14. The alignment mark 70b may be in the form of a cross-hair pattern, as illustrated in FIG. 5 or in some other shape, such as a small circle or ellipse similar to that shown in FIG. 4 for the alignment mark 70a.

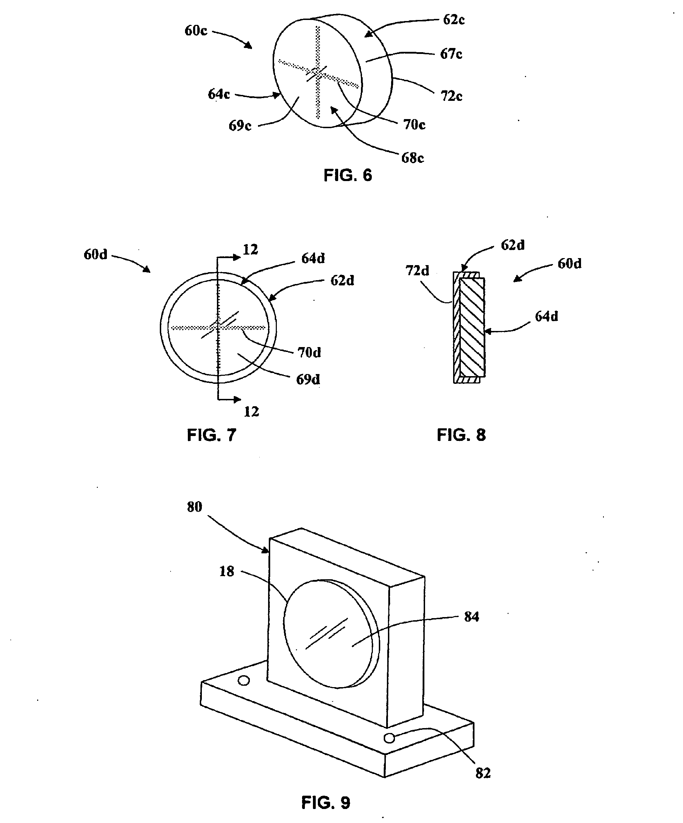

Referring to FIG. 6, in certain embodiments, an alignment tool 60c comprises a base 62c and an alignment optic 64c that are integrally formed from a fluorescent element or material 67c. The alignment optic 64c may be configured similar to the other embodiments described herein. For example, the alignment optic 64c may comprise a front surface 68c including a reflective portion or surface 69c and an alignment mark 70c. One or more of the surfaces of the base 62c may be configured to serve as registration elements 72c when used in conjunction with a mirror within the system 10 that has the same or substantially the same dimensions and/or shape as at least a portion of the base 62c. For example, the alignment tool 60c may be constructed to have the same overall diameter and thickness as one or more of the turning mirrors of the system 10.

Referring to FIGS. 7 and 8, in some embodiments, an alignment tool 60d comprises a housing or base 62d that is configured to hold an alignment optic 64d with a front surface configured according to embodiments of the invention (e.g., including a reflective surface 69d and an alignment mark 70d). The base 62d may also be configured to receive a mirror from the system 10. The housing 62d includes registration elements 72d that may be similar to the registration elements 72c of the previous embodiment. The housing 62d, along with the alignment optic 64d (or system mirror), are inserted or attached to an optical mount disposed within the system 10 for holding and/or aligning the turning mirrors thereof. The housing 62d may be configured to be magnetically coupled to the optical mount. Several housings 62d may be fabricated to hold one or more alignment optics 64d and one or more system 10 mirrors, thus facilitating easy and accurate registration of both system 10 mirrors and alignment optic 64d. In some embodiments, the housing 62d may include a magnetic or similar coupling mechanism that allows the housing 62d to be reliably and repeatably mounted within optical mounts of the system 10.

Referring again to FIGS. 2 and 3, and with additional reference to FIGS. 9-13, the use of the alignment tool 60 in aligning the laser beam deliver system 10 will now be described. It will be appreciated that any of the various alignment tools according embodiments of the invention described or suggested herein, where appropriate, may be used in conjunction with alignment of the system 10. In the forgoing discussion, the alignment tool 60 shown in FIGS. 2 and 3 will be used for illustrative purposes to describe alignment of system 10 turning mirrors. Referencing to FIGS 2 and 3, the alignment optic 64 and/or the base 62 may be configured to at least temporarily replace one or more of the system 10 mirrors and/or mirror fixtures during an alignment procedure. With additional referring to FIG. 9, the first turning mirror 18 may be attached or mounted to a mirror mount, fixture, or base 80 that is configured to receive the first turning mirror 18. The base 80 may comprise one or more registration elements 82 corresponding to the one or more registration elements 72 of the base 62.

In general, the geometry of each of the bases 62, 80 and the registration elements 72, 82 is configured so that the reflective surface 69 of the alignment optic 64 and a reflective surface 84 of turning mirror 18 are disposed in a common plane and with the same angular orientation when either of the bases 62, 80 is mounted in the system 10. During an alignment procedure, the mirror 18 and/or the mirror fixture 74 are temporarily removed from the system 10 and replaced by the base 62 containing the alignment optic 64. The system 10 may then be at least partially aligned using the alignment optic 64 so that at least a portion of the beam 14 is reflected in a predetermined direction by the reflective surface 69 of the alignment optic 64. Once the reflective surface 69 has been properly aligned, the alignment optic 64 may be removed and the turning mirror 18 be re-inserted into the system 10 so that the beam 14 is still reflected in the predetermined direction by the reflective surface 84. In some embodiments, the base 80 may be more securely or permanently coupled to the system 10 and configured to interchangeably hold either the mirror 18 or the alignment optic 64. In such embodiments, either or both the mirror 18 and the alignment optic 64 may be mounted in a housing, such as the housing 62d illustrated in FIGS. 7 and 8, so as to easily and accurately allow interchangeable mounting of the optical elements 18, 64 to the base 80.

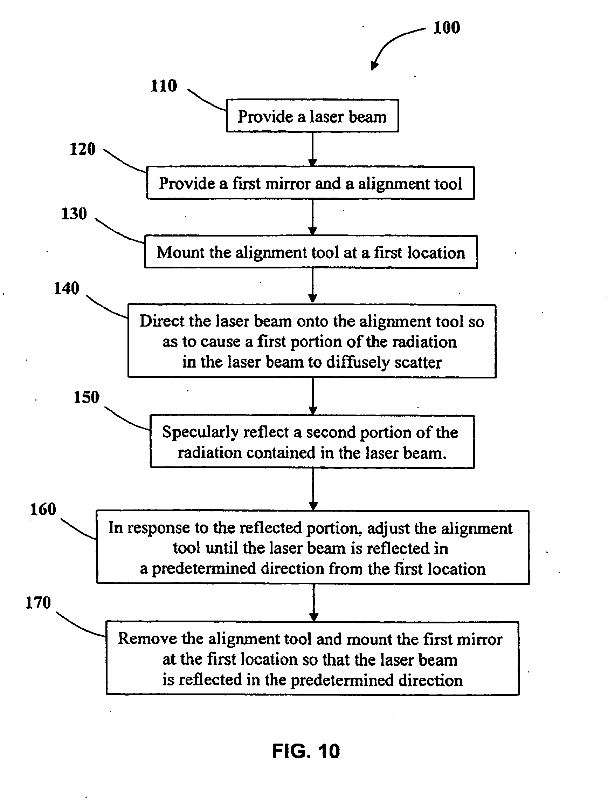

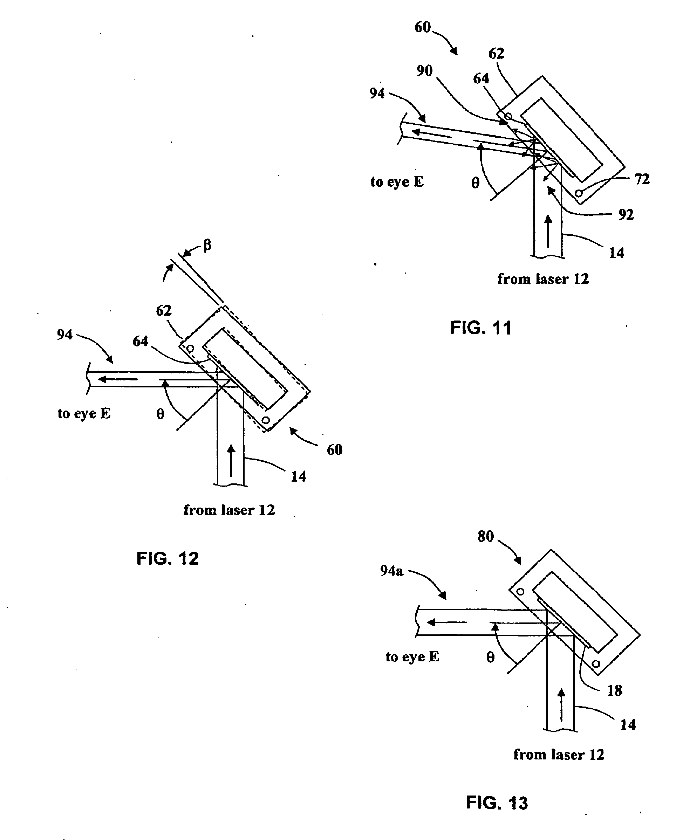

Referring to FIGS. 10 and 11, in certain embodiments, a method 100 of aligning and/or calibrating the laser beam delivery system 10 comprises an operational block 110 of providing the laser beam 14. The method 100 also comprises an operational block 120 of providing the first mirror 18 and the alignment tool 60. The method 100 additionally comprises an operational block 130 of mounting the alignment tool 60 at a first location 90. The method 100 further comprises an operational block 140 of directing the laser beam 14 onto the alignment tool 60 so as to cause a first portion 92 of alignment optic 64 diffusely scatters electromagnetic radiation from the laser beam 14. The method 100 additionally comprises an operational block 150 of causing a second portion 94 of the radiation contained in the laser beam 12 to specularly reflect off the alignment tool 60. The method 100 additionally comprises an operational block 160 of, in response to the reflected portion 94, adjusting the alignment tool 60 until the reflected portion 94 is directed in a predetermined direction θ from the first location 90. The method 100 also comprises an operational block 170 of removing alignment tool 60 and mounting the first mirror 18 at the first location 90 so that the laser beam 12 is reflected in the predetermined θ direction by the mirror 18.

Referring to FIGS. 11-13, the method 100 may be used to align one or more mirrors (e.g., mirrors 18, 20, 40, 42, 48) or other optical components, such as a beamsplitter, within a laser beam deliver system 10. In FIG. 11, the alignment tool 60 is disposed at a first location 90 corresponding to the location of the first mirror 18 within the system 10 (e.g., near the output of the laser 12). The registration elements 72 may be used to insure that the surface 68 of the alignment optic 64 has the same orientation relative to the beam 14 as the surface 84 of the mirror 18.

In certain embodiments, the method 100 additionally or alternatively comprises sensing the laser beam 14 on the alignment tool 60, for example on the front surface 68 and/or within the fluorescent portion 67 (it will be appreciated that the fluorescent portion 67 may include the front surface 68). For example, when the alignment tool 60 is mounted in front of the laser 12 in place of the mirror 18, a footprint of the laser beam 14 may be detected as fluorescent material of the alignment tool 60 fluoresces (e.g., as illustrated in FIG. 4 for the laser footprint 14a). In such embodiments, the method 100 may further comprise, in response to the sensed laser beam, adjusting the location laser beam 14 on the front surface 68, for example, by adjusting one or more translation and/or rotation stages.

In such embodiments, the method 100 may also include, in response to the sensed laser beam, repairing or replacing an optical component of the system 10 or the laser 12 that is found to be defective, for example, based on an abnormal intensity distribution across the footprint formed by the laser beam 14 on the alignment tool 60. For example, if the alignment tool 60 is disposed to replace the turning mirror 18 during an alignment procedure, a defective output coupler (not shown) of the laser 12 may be diagnosed and appropriate action taken to adjust, repair, or replace the output coupler. If the alignment tool 60 is disposed so as to replace the turning mirror 20, then a defect in the turning mirror 18 may similarly be diagnosed and appropriate action taken to adjust, repair, or replace the mirror 18. In similar fashion various upstream optical elements of the system 10 may be inspected to detect defects or damage.

As the beam 14 impinges the alignment tool 60, the reflective surface 69 of the alignment optic 64 reflects the portion 94 of laser beam at the angle θ relative to the surface normal and, for example, may generally direct the beam portion 94 toward the second mirror 20. Some or all of the intermediate elements 22, 24, 28, 30 may be initially removed as the beam 14 is aligned to the second mirror 20. Alternatively, one or more of the elements 22, 24, 28, 30 may be installed, for example, to allow the direction and/or quality of the beam 14 to be assessed, for example, at an intermediate location between the first and second mirrors 18, 20.

In some embodiments, the reflected portion 94 of the laser beam 14 is directed to, and detected on, the next turning mirror in the system (e.g., the second mirror 20 in the present example). In order to aid in the detection of the reflected portion 94, a card or piece of paper containing fluorescent material (e.g., fax paper or thermal paper) may be located at the second mirror 20. In other embodiment of the method 100, the reflected portion 94 is detected at an intermediate location between two turning mirrors (e.g., between the mirrors 18, 20). A fluorescent card or paper may be disposed at the intermediate location for sensing the reflected portion 94. Alternatively or additionally, another alignment tool such as an aperture (e.g., a fixed pinhole or variable iris) may be disposed at the intermediate location. In certain embodiments, two or more apertures may be disposed at different intermediate locations to allow determination of both the location and angular direction of propagation of the reflected portion 94 at the front surface 68 of the alignment optic 64. In such embodiments, the position and/or angle of the front surface 68 may be adjusted to align the laser beam 14 to both apertures. In other embodiments, a photodetector (not shown) intercepts at least a portion of the reflected portion 94. An image of the intercepted portion may be displaced on a monitor for visual inspection and/or a signal may be sent to a computer or micro-processor system for analysis. The resulting visual inspection and/or analysis may be used to determine the location of the reflected beam (for alignment purposes) or to assess the beam quality. The resulting information may be used to support automated or semi-automated alignment of the system 10 or portions thereof.

In some embodiments, the alignment tool 60 is mounted to a rotation stage to which the mirror mount 80 is attached during normal operation of the system 10; wherein the rotation stage may be used adjust the angle of the reflected portion 90. This adjustment in the reflected beam angle is illustrated using FIGS. 11 and 12, in which the angle θ is decreased between FIG. 11 and FIG. 12, relative to a surface normal of the optical element. In this example, the alignment tool 60 is rotated an angle β to change the reflected angle θ of the reflected portion 94 (the dotted lines in FIG. 12 show the approximate orientation of the alignment tool 60 prior to the angular adjustment). Once the angle θ has been adjusted to a desired value, the alignment tool 60 may be removed and the mirror mount 80 and the mirror 18 put back into the system 10, for example, using the registration elements 82 of the mirror mount 80 to insure that the surface of the surface of the mirror 18 has the same orientation relative to the beam 14 as the alignment optic 64. Alternatively, the alignment tool 60 may remain in the system 10 while other components of the system 10 are aligned.

In certain embodiments, the method 100 is implemented using the alignment tools 60c or 60d. In such embodiments, the alignment tool 60c or 60d may be mounted directly to the mirror mount 80 during alignment of the system 10 and then replaced by the mirror 18 (for example) during normal operation or during a later stage in the alignment procedure. The mirror mount 80, the mirror 18 (for example), and the alignment optic 64 are configured so that reflective surface of either element 18, 64 has the same orientation when placed within the mirror mount 80. Thus, any adjustments to the mirror mount 80 will affect the reflected angle θ the same way, regardless of whether the alignment optic 64 or the mirror 18 are mounted within the mirror mount 80.

Once the alignment of the first mirror 18 has been completed, the second mirror 20 (or some other component of the system 10) may be replaced by the alignment tool 60 (or a second alignment tool 60) to continue the alignment procedure according to the method 100 or similar such method. Alternatively, one or more of the mirrors of the system 10 may be at least temporally replaced by the alignment optic 64, or similar such optical element, by removing the system mirror from its mount and inserting the alignment optic 64 in its place. In some embodiments, alignment of the system 10 is carried out using two alignment tools 60 (or alignment optical elements 64) that are simultaneously disposed at different locations within the system 10 (e.g., at the locations of the first and second mirrors 18, 20). In any event, the method 100 may further comprise adjusting the rotation and/or location of the alignment tool 60 at the new location, and/or one or more additional alignment tools or alignment optical elements disposed at the new location, to align the system 10 (for example, to align the system 10 to the ablation plane 50).

The above presents a description of the best mode contemplated of carrying out the present invention, and of the manner and process of making and using it, in such full, clear, concise, and exact terms as to enable any person skilled in the art to which it pertains to make and use this invention. This invention is, however, susceptible to modifications and alternate constructions from that discussed above. Consequently, it is not the intention to limit this invention to the particular embodiments disclosed. On the contrary, the intention is to cover modifications and alternate constructions coming within the spirit and scope of the invention as generally expressed by the following claims, which particularly point out and distinctly claim the subject matter of the invention.

## Claims

1. A laser beam delivery system (10) for an ocular surgery, comprising:
a laser (12) configured to produce a beam of electromagnetic radiation;
a first mirror (18); and
a first alignment optic (60), comprising:
a fluorescent element (67) configured to fluoresce when illuminated by electromagnetic radiation produced by the laser; and
a front surface (68) comprising a reflective surface (69), the reflective surface configured to specularly reflect at least a portion of the laser beam; and
a first site configured to removably receive either one of the first mirror and the first alignment optic, so that at least a portion of the laser beam reflects in a predetermined direction;
**characterised in that**
the front surface comprises an alignment mark (70), and the alignment mark is centrally disposed within the reflective surface and configured to diffusely scatter at least some of the electromagnetic radiation of the laser beam.

2. The delivery system of claim 1, further comprising a system mount disposed at the site configured to removably receive either one of the alignment optic and the mirror.

3. The delivery system of claim 2, further comprising a first optical mount for holding the mirror and a second optical mount (62) for holding the alignment optic, the site configured to removably receive either one of the first and second optical mounts.

4. The delivery system of claim 1, further comprising a second mirror and a second site configured to receive either one of the alignment optic and the second mirror.

5. The delivery system of claim 1, further comprising a second mirror (20), a second alignment optic, and a second site, the second site configured to removably receive either one of the second mirror and the second alignment optic.

6. A method of aligning a laser beam delivery system (10) for an ocular surgery, the method comprising:
providing a laser beam (12);
providing a first mirror (18) and an alignment tool (60), the alignment tool comprising:
a fluorescent element (67) configured to fluoresce when illuminated by electromagnetic radiation produced by the laser; and
a front surface (68) comprising a reflective surface (69), the reflective surface configured to specularly reflect the electromagnetic radiation;
mounting the alignment tool at a first location; directing the laser beam onto the alignment tool so as to cause a first portion of the radiation in the laser beam to diffusely scatter and a second portion of the radiation in the laser beam to specularly reflect;
in response to the reflected portion, adjusting the alignment tool until the portion of the laser beam is reflected in a predetermined direction from the first location; and
removing the alignment tool and mounting the first mirror at the first location so that the laser beam is reflected in the predetermined direction by the mirror;
**characterised in that**
the front surface comprises an alignment mark (70), and the alignment mark is centrally disposed within the reflective surface and configured to diffusely scatter the electromagnetic radiation.

7. The method of claim 6, further comprising:
mounting the alignment tool at a second location;
directing the laser beam onto the alignment tool at the second location so as to specularly reflect at least a second portion of the radiation contained in the laser beam;
in response to the second reflected portion, adjusting the alignment tool until the laser beam is reflected in a second predetermined direction; and
removing alignment tool and mounting a second mirror (20) at the second location so that the laser beam is reflected in the second predetermined direction by the second mirror.

8. The method of claim 6, further comprising:
mounting a second alignment tool at a second location;
directing the laser beam onto the second alignment tool so as to specularly reflect at least a second portion of the radiation contained in the laser beam;
in response to the second reflected portion, adjusting at least one of the second alignment tool the reflected portion from the first location until the laser beam is reflected in a second predetermined direction;
removing alignment tool and mounting a second mirror at the second location so that the laser beam is reflected in the second predetermined direction by the second mirror.

9. An alignment tool (60) for a laser beam delivery system, comprising:
an alignment optic operably coupled to the base, the alignment optic comprising:
a fluorescent element (67) configured to fluoresce when illuminated by electromagnetic radiation produced by a laser; and
a front surface (68) comprising a reflective surface (69), the reflective surface configured to specularly reflect the electromagnetic radiation
**characterised in that**
the tool comprises a base (62) including one or more registration elements (72), the front surface comprises an alignment mark (70), and the alignment mark is centrally disposed within the reflective surface and configured to diffusely scatter the electromagnetic radiation.

10. The alignment tool of claim 9, wherein the alignment mark comprises horizontal and vertical lines, the lines intersecting at the center of the reflective surface.

11. The alignment tool of claim 9, wherein the alignment mark comprises a circle that is centrally disposed within the reflective surface.

12. The alignment tool of claim 9, wherein the base comprises an optical mount for holding the alignment optic.

13. The alignment tool of claim 9, wherein the reflective surface comprises a material that is disposed on top of the fluorescent element.

14. The alignment tool of claim 9, wherein the alignment mark is disposed at the center of the reflective surface, the area of the reflective surface being at least about 25 times greater than the area of the alignment mark.

15. The delivery system of claim 1 or the alignment tool of claim 9, wherein the electromagnetic radiation is within an ultraviolet band or an infrared band of the electromagnetic spectrum.

## Patentansprüche

1. Laserstrahlzuführsystem (10) für eine Augenoperation, mit:
einem Laser (12), der eingerichtet ist, einen Strahl elektromagnetischer Strahlung zu erzeugen;
einem ersten Spiegel (18); und
einer ersten Ausrichtungsoptik (60), mit:
einem Fluoreszenzelement (67), das eingerichtet ist, zu fluoreszieren, wenn es durch eine durch den Laser erzeugte elektromagnetische Strahlung beleuchtet wird; und
einer Vorderfläche (68) mit einer reflektierenden Fläche (69), die eingerichtet ist, zumindest einen Abschnitt des Laserstrahls spiegelnd zu reflektieren; und
einem ersten Ort, der eingerichtet ist, den ersten Spiegel oder die erste Ausrichtungsoptik abnehmbar aufzunehmen, sodass zumindest ein Abschnitt des Laserstrahls in einer festgelegten Richtung reflektiert;
**dadurch gekennzeichnet, dass**
die Vorderfläche eine Ausrichtungsmarkierung (70) aufweist, die mittig innerhalb der reflektierenden Fläche angeordnet ist und eingerichtet ist, zumindest einen Teil der elektromagnetischen Strahlung des Laserstrahls diffus zu streuen.

2. Zuführsystem nach Anspruch 1, des Weiteren mit einer Systemhalterung, die an dem Ort angeordnet ist, der eingerichtet ist, die Ausrichtungsoptik oder den Spiegel abnehmbar aufzunehmen.

3. Zuführsystem nach Anspruch 2, des Weiteren mit einer ersten optischen Halterung zum Halten des Spiegels und einer zweiten optischen Halterung (62) zum Halten der Ausrichtungsoptik, wobei der Ort eingerichtet ist, die erste oder die zweite optische Halterung abnehmbar aufzunehmen.

4. Zuführsystem nach Anspruch 1, des Weiteren mit einem zweiten Spiegel und einem zweiten Ort, der eingerichtet ist, die Ausrichtungsoptik oder den zweiten Spiegel aufzunehmen.

5. Zuführsystem nach Anspruch 1, des Weiteren mit einem zweiten Spiegel (20), einer zweiten Ausrichtungsoptik und einem zweiten Ort, der eingerichtet ist, den zweiten Spiegel oder die zweite Ausrichtungsoptik abnehmbar aufzunehmen.

6. Verfahren zum Ausrichten eines Laserstrahlzuführsystems (10) für eine Augenoperation, das Verfahren mit:
Bereitstellen eines Laserstrahls (12);
Bereitstellen eines ersten Spiegels (18) und eines Ausrichtungsinstruments (60), das Ausrichtungsinstrument mit:
einem Fluoreszenzelement (67), das eingerichtet ist, zu fluoreszieren, wenn es durch mit dem Laser erzeugte elektromagnetische Strahlung beleuchtet wird; und
einer Vorderfläche (68) mit einer reflektierenden Fläche (69), die eingerichtet ist, die elektromagnetische Strahlung spiegelnd zu reflektieren;
Montieren des Ausrichtungsinstruments an einer ersten Stelle;
Ausrichten des Laserstrahls auf das Ausrichtungsinstrument, um zu verursachen, dass ein erster Abschnitt der Strahlung in dem Laserstrahl diffus gestreut wird und ein zweiter Abschnitt der Strahlung in dem Laserstrahl spiegelnd reflektiert wird;
Einstellen des Ausrichtungsinstruments als Antwort auf den reflektierten Abschnitt bis der Abschnitt des Laserstrahls in einer festgelegten Richtung von der ersten Stelle reflektiert wird; und
Entfernen des Ausrichtungsinstruments und Montieren des ersten Spiegels an der ersten Stelle, sodass der Laserstrahl durch den Spiegel in der festgelegten Richtung reflektiert wird;
**dadurch gekennzeichnet, dass**
die Vorderfläche eine Ausrichtungsmarkierung (70) aufweist, die mittig in der reflektierenden Fläche angeordnet ist und eingerichtet ist, die elektromagnetische Strahlung diffus zu streuen.

7. Verfahren nach Anspruch 6, des Weiteren mit:
Montieren des Ausrichtungsinstruments an einer zweiten Stelle;
Ausrichten des Laserstrahls auf das Ausrichtungsinstrument an der zweiten Stelle, um so zumindest einen zweiten Abschnitt der in dem Laserstrahl enthaltenen Strahlung spiegelnd zu reflektieren;
Einstellen des Ausrichtungsinstruments als Antwort auf den zweiten reflektierten Abschnitt, bis der Laserstrahl in einer zweiten festgelegten Richtung reflektiert wird; und
Entfernen des Ausrichtungsinstruments und Montieren eines zweiten Spiegels (20) an der zweiten Stelle, sodass der Laserstrahl durch den zweiten Spiegel in der zweiten festgelegten Richtung reflektiert wird.

8. Verfahren nach Anspruch 6, des Weiteren mit:
Montieren eines zweiten Ausrichtungsinstruments an einer zweiten Stelle;
Ausrichten des Laserstrahls auf das zweite Ausrichtungsinstrument, um so zumindest einen zweiten Abschnitt der in dem Laserstrahl enthaltenden Strahlung spiegelnd zu reflektieren;
als Antwort auf den zweiten reflektierten Abschnitt, Einstellen zumindest der reflektierte Abschnitt von der ersten Stelle bis der Laserstrahl in einer zweiten festgelegten Richtung reflektiert wird;
Entfernen des Ausrichtungsinstruments und Montieren eines zweiten Spiegels an der zweiten Stelle, sodass der Laserstrahl durch den zweiten Spiegel in der zweiten festgelegten Richtung reflektiert wird.

9. Ausrichtungsinstrument (60) für ein Laserstrahlzuführsystem, mit:
einer Ausrichtungsoptik, die funktionsfähig mit der Basis gekoppelt ist, mit:
einem Fluoreszenzelement (67), das eingerichtet ist, zu fluoreszieren, wenn es durch mit einem Laser erzeugte elektromagnetische Strahlung beleuchtet wird; und
einer Vorderfläche (68) mit einer reflektierenden Fläche (69), die eingerichtet ist, die elektromagnetische Strahlung spiegelnd zu reflektieren,
**dadurch gekennzeichnet, dass**
das Instrument eine Basis (62) aufweist, die ein oder mehrere Erfassungselemente (72) aufweist, die Vorderfläche eine Ausrichtungsmarkierung (70) aufweist und die Ausrichtungsmarkierung mittig in der reflektierenden Fläche angeordnet ist und eingerichtet ist, die elektromagnetische Strahlung diffus zu streuen.

10. Ausrichtungsinstrument nach Anspruch 9, bei dem die Ausrichtungsmarkierung eine horizontale und eine vertikale Linie umfasst, wobei die Linien sich in der Mitte der reflektierenden Fläche schneiden.

11. Ausrichtungsinstrument nach Anspruch 9, bei dem die Ausrichtungsmarkierung einen Kreis aufweist, der mittig in der reflektierenden Fläche angeordnet ist.

12. Ausrichtungsinstrument nach Anspruch 9, bei dem die Basis eine Optikhalterung zum Halten der Ausrichtungsoptik aufweist.

13. Ausrichtungsinstrument nach Anspruch 9, bei dem die reflektierende Fläche ein Material aufweist, das auf dem Fluoreszenzelement angeordnet ist.

14. Ausrichtungsinstrument nach Anspruch 9, bei dem die Ausrichtungsmarkierung in der Mitte der reflektierenden Fläche angeordnet ist, wobei die Fläche der reflektierenden Fläche mindestens 25 Mal größer ist als die Fläche der Ausrichtungsmarkierung.

15. Zuführsystem nach Anspruch 1 oder das Ausrichtungsinstrument nach Anspruch 9, wobei die elektromagnetische Strahlung in einem ultravioletten Bereich oder einem Infrarotbereich des elektromagnetischen Spektrums ist.

## Revendications

1. Système (10) d'émission de faisceau laser pour une chirurgie oculaire, comprenant :
un laser (12) configuré pour produire un faisceau de rayonnement électromagnétique ;
un premier miroir (18) ; et
un premier élément optique d'alignement (60), comprenant :
un élément fluorescent (67) configuré pour fluorescer lorsqu'il est éclairé par un rayonnement électromagnétique produit par le laser ; et
une surface avant (68) comprenant une surface réfléchissante (69), la surface réfléchissante configurée pour réfléchir de manière spéculaire au moins une partie du faisceau laser ; et
un premier site configuré pour recevoir de manière amovible l'un quelconque du premier miroir et du premier élément optique d'alignement, de sorte qu'au moins une partie du faisceau laser réfléchisse dans une direction prédéterminée ;
**caractérisé en ce que**
la surface avant comprend une marque d'alignement (70), et la marque d'alignement est disposée centralement dans la surface réfléchissante et configurée pour disperser de manière diffuse au moins une partie du rayonnement électromagnétique du faisceau laser.

2. Système d'émission de la revendication 1, comprenant en outre une monture du système disposée au niveau du site configurée pour recevoir de manière amovible l'un quelconque de l'élément optique d'alignement et du miroir.

3. Système d'émission de la revendication 2, comprenant en outre une première monture optique destinée à maintenir le miroir et une deuxième monture optique (62) destinée à maintenir l'élément optique d'alignement, le site configuré pour recevoir de manière amovible l'une quelconque des première et deuxième montures optiques.

4. Système d'émission de la revendication 1, comprenant en outre un deuxième miroir et un deuxième site étant configuré pour recevoir l'un quelconque de l'élément optique d'alignement et du deuxième miroir.

5. Système d'émission de la revendication 1, comprenant en outre un deuxième miroir (20), un deuxième élément optique d'alignement, et un deuxième site, le deuxième site configuré pour recevoir de manière amovible l'un quelconque du deuxième miroir et du deuxième élément optique d'alignement.

6. Procédé d'alignement d'un système (10) d'émission de faisceau laser pour une chirurgie oculaire, le procédé comprenant le fait :
de fournir un faisceau laser (12) ;
de fournir un premier miroir (18) et un outil d'alignement (60), l'outil d'alignement comprenant :
un élément fluorescent (67) configuré pour fluorescer lorsqu'il est éclairé par un rayonnement électromagnétique produit par le laser ; et
une surface avant (68) comprenant une surface réfléchissante (69), la surface réfléchissante configurée pour réfléchir de manière spéculaire le rayonnement électromagnétique ;
de monter l'outil d'alignement à un premier emplacement ; de diriger le faisceau laser sur l'outil d'alignement de manière à amener une première partie du rayonnement dans le faisceau laser à disperser de manière diffuse et une deuxième partie du rayonnement dans le faisceau laser à réfléchir de manière spéculaire ;
de régler l'outil d'alignement, en réponse à la partie réfléchie, jusqu'à ce que la partie du faisceau laser soit réfléchie dans une direction prédéterminée à partir du premier emplacement ; et
d'enlever l'outil d'alignement et de monter le premier miroir au premier emplacement de sorte que le faisceau laser soit réfléchi dans la direction prédéterminée par le miroir ;
**caractérisé en ce que**
la surface avant comprend une marque d'alignement (70), et la marque d'alignement est disposée centralement dans la surface réfléchissante et configurée pour disperser de manière diffuse le rayonnement électromagnétique.

7. Procédé de la revendication 6, comprenant en outre le fait :
de monter l'outil d'alignement à un deuxième emplacement ;
de diriger le faisceau laser sur l'outil d'alignement au niveau du deuxième emplacement de manière à réfléchir de manière spéculaire au moins une deuxième partie du rayonnement contenu dans le faisceau laser ;
de régler l'outil d'alignement, en réponse à la deuxième partie réfléchie, jusqu'à ce que le faisceau laser soit réfléchi dans une deuxième direction prédéterminée ; et
d'enlever l'outil d'alignement et de monter un deuxième miroir (20) au niveau du deuxième emplacement de sorte que le faisceau laser soit réfléchi dans la deuxième direction prédéterminée par le deuxième miroir.

8. Procédé de la revendication 6, comprenant en outre le fait :
de monter un deuxième outil d'alignement à un deuxième emplacement ;
de diriger le faisceau laser sur le deuxième outil d'alignement de manière à réfléchir de manière spéculaire au moins une deuxième partie du rayonnement contenu dans le faisceau laser ;
en réponse à la deuxième partie réfléchie, de régler au moins l'un du deuxième outil d'alignement la partie réfléchie à partir du premier emplacement jusqu'à ce que le faisceau laser soit réfléchi dans une deuxième direction prédéterminée ;
d'enlever l'outil d'alignement et de monter un deuxième miroir au deuxième emplacement de sorte que le faisceau laser soit réfléchi dans la deuxième direction prédéterminée par le deuxième miroir.

9. Outil d'alignement (60) pour un système de d'émission de faisceau laser, comprenant :
un élément optique d'alignement couplé de manière fonctionnelle à la base, l'élément optique d'alignement, comprenant :
un élément fluorescent (67) configuré pour fluorescer lorsqu'il est éclairé par un rayonnement électromagnétique produit par un laser ; et
une surface avant (68) comprenant une surface réfléchissante (69), la surface réfléchissante configurée pour réfléchir de manière spéculaire le rayonnement électromagnétique,
**caractérisé en ce que**
l'outil comprend une base (62) comportant un ou plusieurs éléments d'enregistrement (72), la surface avant comprend une marque d'alignement (70), et la marque d'alignement est disposée centralement dans la surface réfléchissante et configurée pour disperser de manière diffuse le rayonnement électromagnétique.

10. Outil d'alignement de la revendication 9, dans lequel la marque d'alignement comprend des lignes horizontales et verticales, les lignes se coupant au centre de la surface réfléchissante.

11. Outil d'alignement de la revendication 9, dans lequel la marque d'alignement comprend un cercle qui est disposé centralement dans la surface réfléchissante.

12. Outil d'alignement de la revendication 9, dans lequel la base comprend une monture optique destinée à maintenir l'élément optique d'alignement.

13. Outil d'alignement de la revendication 9, dans lequel la surface réfléchissante comprend un matériau qui est disposé au sommet de l'élément fluorescent.

14. Outil d'alignement de la revendication 9, dans lequel la marque d'alignement est disposée au centre de la surface réfléchissante, l'aire de la surface réfléchissante étant au moins environ 25 fois plus grand que l'aire de la marque d'alignement.

15. Système de délivrance de la revendication 1 ou l'outil d'alignement de la revendication 9, dans lequel le rayonnement électromagnétique se situe dans une bande ultraviolette ou une bande infrarouge du spectre électromagnétique du spectre électromagnétique.
